# EUROPEAN PATENT APPLICATION

(11) **EP 1 752 149 A1**
(43) Date of publication of application: **14.02.2007**
(21) Application number: 05384035.1
(22) Date of filing: 29.07.2005
(51) Int. Cl.: A61K 31/437, A61P 9/10, A61P 29/00

(54) **CB1 Antagonists or inverse agonists as therapeutical agents for the treatment of inflammation involving gene expression**

(71) Applicant: LABORATORIOS DEL DR. ESTEVE, S.A., 08041 Barcelona (ES)
(72) Inventor: Buschmann, Helmut Heinrich, 08960 Sant Just Desvern (Barcelona) (ES)

(57) **Abstract**

The present invention relates to substituted pyrazoline compounds, methods for their preparation, medicaments comprising these compounds as well as their use for the preparation of a medicament for the treatment of humans and animal.

## Description

The present invention relates to CB1 Antagonists or Inverse Agonists as therapeutical agents for the treatment of inflammation involving gene expression.

Inflammation is a serious healthproblem and can - especially in inflammatory heart diseases - become possibly very threatening. Therefore there is a constant need for treatment of this disease states.

Thus, it was an object of the present invention to provide novel compounds for use as active substances in medicaments for the treatment of inflammation.

Thus an aspect of the current invention is the use of a CB1 antagonist or inverse agonist for the production of a medicament for the treatment and/or prophylaxis of an inflammation that involves expression of genes involved in inflammation and/or in the immune response. Possible candidates for the expressed genes include cytokines (IL-2, IL-6 and TNFα), COX-2 and iNOS.

Another closely related aspect of the current invention is also the use of CB1 antagonists or inverse agonists for the manufacture of a medicament for the treatment and/or prophylaxis of an inflammation based on an immunologic effect.

It was surprisingly found that Canabinoid Receptor Inverse Agonists and Antagonists are able to inhibit transcription of genes involved in the inflammatory process and in the immune response and thus act as anti-inflammatory agents.

Consequently another aspect of the invention is also the use of CB1 antagonists or inverse agonists for the manufacture of a medicament for the treatment and/or prophylaxis of immunologic disorders that involve expression of genes involved in inflammation and/or in the immune response.

As a further consequence thereof in addition another aspect of the invention is also the use of a CB1 antagonist or inverse agonist for the production of a medicament for the manufacture of a medicament for the treatment and/or prophylaxis of inflammatory heart diseases, especially those that involve expression of genes involved in inflammation and/or in the immune response. Examples of inflammatory heart diseases are angina pectoris and artherio sclerosis, whereas for the latter the invention preferably refers to the prophylaxis of artherio sclerosis.

Another preferred aspect of the invention is also a method of treatment encompassing all the abovementioned uses, wherein the CB1 antagonist or inverse agonist is applied to a person in need thereof, to treat- using its immunomodulary or anti-inflammatory aspects - inflammation or immunologic disorders.

In an embodiment the use of the canabinoid receptor agonist or inverse agonist, especially according to general formula I, in the production of a medicament for the treatment of sclerotic plaque is disclaimed.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992. The term "CB1 antagonist or inverse agonists" in the sense of this invention is defined as a compound which binds to the CB1 receptor and has the functionality of an antagonist or inverse agonist. A CB1 inverse agonist both blocks the action of an agonist and attenuates receptor constitutive activity. A CB1 antagonist blocks the action of the agonist but is ineffective on the receptor-constitutive activity. For an overview of the destinction see e.g. R Pertwee, Esteve Foundation Symposia Vol. 10, 75 (2003). Canabinoid receptor ligands may be functionally characterized, for example, according to:
(1) their effect upon adenyly cyclase activity; and/or
(2) their effect upon [³⁵S]-g-GTP binding.

An inverse agonist will stimulate (1)adenylyl cyclase activity and (2) will inhibit [³⁵S]-g-GTP binding.
An antagonist will (1) block the inhibition of adenylyl cyclase activity by CB1 agonists and (2) block the stimulation of [³⁵S]-g-GTP binding by a CB1 agonist.

For more details and a description of the tests to distinguish the compounds reference is made to WO2004/078261 A1, pages 20 to 28 included here entirely by reference, especially in regards to the EC50 and IC 50 values and Kb values, the criteria and the tests.
In one embodiment the CB1 antagonist does not effect the cAMP production, but blocks the inhibition of cyclic AMP production by a canabinoid receptor agonist with a a Kb value of 10 µM or less (e.g. 1 µM, 500 nM, 200 nM, 100 nM, 50 nM, 20 nM 10 nM, 5nM).
In one embodiment the CB1 antagonist does not effect the [³⁵S]-g-GTP binding, but blocks the stimulation of [³⁵S]-g-GTP binding by a canabinoid receptor agonist with a a Kb value of 10 µM or less (e.g. 1 µM, 500 nM, 200 nM, 100 nM, 50 nM, 20 nM 10 nM, 5nM).
In one embodiment both of the above appliy.

In one embodiment the CB1 inverse agonist stimulates cAMP production with an EC50 value of 10 mM or less (e.g. 1 µM, 500 nM, 200 nM, 100 nM, 50 nM, 20 nM, 10 nM, 5nM).

In one embodiment the CB1 inverse agonist inhibits the [³⁵S]-g-GTP binding, with an IC50 value of 10 µM or less (e.g. 1 µM, 500 nM, 200 nM, 100 nM, 50 nM, 20 nM 10 nM, 5nM).
In one embodiment both of the above appliy.

In addition it is referred to the following articles on CB1 antagonists included in the description by reference completely but also added as ANNEXES on pages: 72 to 80 and 81 to 94 respectively of this application.
**ANNEX 1:**
   Lange and Kruse, "Recent advances in CB1 cannabinoid receptor antagonists", Current Opinion in Drug Discovery & Development, 2004, 7(4): 498-506
**ANNEX 2:**
   Smith and Fathi, "Recent advances in the research and development of CB1 antagonists", IDrugs 2005 8(1):53-66

In another preferred embodiment of the invention the CB1 Antagonist or Inverse Agonist used according to the invention is selected from a compound of general formula I 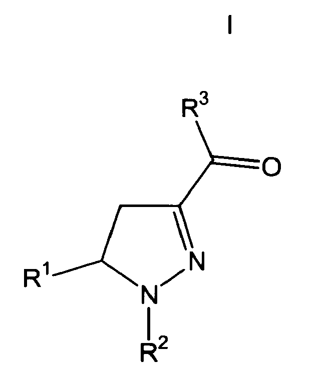 wherein
R¹ represents an optionally at least mono-substituted phenyl group,
R² represents an optionally at least mono-substituted phenyl group,
R³ represents a saturated or unsaturated, optionally at least mono-substituted, optionally at least one heteroatom as ring member containing cycloaliphatic group, which may be condensed with an optionally at least mono-substituted mono- or polycyclic ring system, or R³ represents an optionally at least mono-substituted aryl or heteroaryl group, which may be condensed with an optionally at least mono-substituted mono- or polycyclic ring system, or R³ represents an ―NR⁴R⁵-moiety,
R⁴ and R⁵, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R⁶-moiety, or an -NR⁷R⁸-moiety,
R⁶ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R⁷ and R⁸, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

It has been found that these substituted pyrazoline compounds of general formula I given below, their stereoisomers, corresponding salts and corresponding solvates thereof. have a high affinity for cannabinoid receptors, particularly for the CB₁-receptor, and that they act as modulators e.g. antagonists and inverse agonists on these receptors. They are therefore suitable for the prophylaxis and/or treatment of inflammation in humans and/or animals, preferably humans including infants, children and grown-ups.

Particularly preferably the following provisos (disclaimers) apply for the pyrazoline compounds of general formula I given above:
that R⁴ and R⁵ do not both represent a hydrogen atom, and
that if one of the residues R⁴ and R⁵ represents a hydrogen atom or an alkyl group, which is optionally at least mono-substituted with an alkoxy group, an alkoxyalkoxy group, a halogen atom or a phenyl group, the other one of these residues R⁴ and R⁵ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an -OCH₂O-, -OCH₂CH₂O- or -CH₂CH₂O- chain, which is optionally substituted with one or more halogen atoms or one or two methyl groups, an -NH-phenyl-moiety, wherein the phenyl group may be mono-substituted in the 4-position, and
that if one of the residues R⁴ and R⁵ represents an alkynyl group, the other one of these residues R⁴ and R⁵ does not represent a phenyl group, which is optionally substituted in the 4-position, and
that if one of the residues R⁴ and R⁵ represents a hydrogen atom or a linear or branched, saturated or unsaturated, unsubstituted or substituted aliphatic radical, the other one of these residues R⁴ and R⁵ does not represent an unsubstituted or substituted thiazole group or an unsubstituted or substituted [1,3,4]thiadiazole group.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues R³-R⁸ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched C₁₋₆-alkoxy, branched or unbranched C₁₋₆-alkyl, branched or unbranched C₁₋₄-perfluoroalkoxy, branched or unbranched C₁₋₄-perfluoroalkyl, oxo, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl , wherein the C₁₋₄-alkyl may in each case be branched or unbranched, and a phenyl group, more preferably be selected from the group consisting of hydroxy, F, Cl, Br, methyl, ethyl, methoxy, ethoxy, oxo, CF₃ and a phenyl group.

If one or more of the residues R³-R⁸ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues R³―R⁸ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched C₁₋₆-alkoxy, branched or unbranched C₁₋₆-alkyl, branched or unbranched C₁₋₄-perfluoroalkoxy, branched or unbranched C₁₋₄-perfluoroalkyl, amino, carboxy, oxo, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl , wherein the C₁₋₄-alkyl may in each case be branched or unbranched, and a phenyl group, more preferably be selected from the group consisting of hydroxy, F, Cl, Br, methyl, ethyl, methoxy, ethoxy, CF₃, oxo and a phenyl group.

If one or more of the residues R¹-R⁸ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched C₁₋₆-alkyl group, a linear or branched C₁₋₆ alcoxy group, a formyl group, a hydroxy group, a trifluoromethyl group, a trifluoromethoxy group, a -CO-C₁₋₆-alkyl group, a cyano group, a nitro group, a carboxy group, a -CO-O-C₁₋₆-alkyl group, a -CO-NR^{A}R^{B}- moiety, a -CO-NH-NR^{C}R^{D}-moiety, an -SH, an -S-C₁₋₆-alkyl group, an -SO-C₁-₆-alkyl group, an -SO₂-C₁₋₆-alkyl group, a -C₁₋₆-alkylene-S-C₁₋₆-alkyl group, a -C₁₋₆-alkylene-SO-C₁₋₆-alkyl group, a -C₁₋₆-alkylene-SO₂-C₁₋₆-alkyl group, an -NH₂-moiety, an NHR'-moiety or an NR'R"-moiety, wherein R' and R" independently represent a linear or branched C₁₋₆-alkyl group, a C₁₋₆-alkyl group substituted by one or more hydroxy groups and a ―C₁₋₆-alkylene-NR^{E}R^{F} group,
whereby R^{A}, R^{B}, identical or different, represent hydrogen or a C₁₋₆-alkyl group, or R^{A} and R^{B} together with the bridging nitrogen atom form a saturated, mono- or bicyclic, 3-10 membered heterocyclic ring system, which may be at least mono-substituted by one or more, identical or different, C₁₋₆ alkyl groups and/or which may contain at least one further heteroatom selected from the group consisting of nitrogen, oxygen and sulphur as a ring member,
R^{C}, R^{D}, identical or different, represent a hydrogen atom, a C₁₋₆-alkyl group, a -CO-O-C₁₋₆-alkyl group, a C₃₋₈-cycloalkyl group, a C₁₋₆-alkylene-C₃₋₈-cycloalkyl group, C₁₋₆-alkylene-O-C₁₋₆-alkyl group or a C₁₋₆-alkyl group substituted with one or more hydroxy groups, or R^{C}, R^{D} together with the bridging nitrogen atom form a saturated, mono- or bicyclic, 3-10 membered heterocyclic ring system, which may be at least mono-substituted by one or more substituents independently selected from the group consisting of C₁₋₆ alkyl group, a -CO-C₁₋₆-alkyl group, a ―CO-O- C₁₋₆-alkyl group, a -CO-NH- C₁₋₆-alkyl group, a -CS-NH- C₁₋₆-alkyl group, an oxo group, a C₁₋₆-alkyl group substituted with one or more hydroxy groups, a C₁₋₆-alkylene-O-C₁₋₆-alkyl group and a ―CO-NH₂ group and/or which may contain at least one further heteroatom selected from the group consisting of nitrogen, oxygen and sulphur as a ring member, and
wherein R^{E}, R^{F}, identical or different, represent hydrogen or a C₁₋₆-alkyl group, or R^{E} and R^{F} together with the bridging nitrogen atom form a saturated, mono- or bicyclic, 3-10 membered heterocyclic ring system, which may be at least mono-substituted by one or more, identical or different C₁₋₆ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues R³-R⁸ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched C₁₋₆-alkyl group, a linear or branched C₁₋₆ alcoxy group, a formyl group, a hydroxy group, a trifluoromethyl group, a trifluoromethoxy group, a -CO-C₁₋₆-alkyl group, a cyano group, a carboxy group, a ―CO-O-C₁₋₆-alkyl group, a -CO-NR^{A}R^{B}- moiety, a -CO-NH-NR^{C}R^{D}-moiety, an -S-C₁₋₆-alkyl group, an -SO-C₁₋₆-alkyl group, an -SO₂-C₁₋₆-alkyl group, a -C₁₋₆-alkylene-S-C₁₋₆-alkyl group, a -C₁₋₆-alkylene-SO-C₁₋₆-alkyl group, a -C₁₋₆-alkylene-SO₂-C₁₋₆-alkyl group, a C₁₋₆-alkyl group substituted by one or more hydroxy groups and a ―C₁₋₆-alkylene-NR^{E}R^{F} group,
whereby R^{A}, R^{B}, identical or different, represent hydrogen or a C₁₋₆-alkyl group, or R^{A} and R^{B} together with the bridging nitrogen atom form a saturated, mono- or bicyclic, 3-10 membered heterocyclic ring system, which may be at least mono-substituted by one or more, identical or different, C₁₋₆ alkyl groups and/or which may contain at least one further heteroatom selected from the group consisting of nitrogen, oxygen and sulphur as a ring member,
R^{C}, R^{D}, identical or different, represent a hydrogen atom, a C₁₋₆-alkyl group, a -CO-O-C₁₋₆-alkyl group, a C₃₋₈-cycloalkyl group, a C₁₋₆-alkylene-C₃₋₈-cycloalkyl group, C₁₋₆-alkylene-O-C₁₋₆-alkyl group or a C₁₋₆-alkyl group substituted with one or more hydroxy groups, or R^{C}, R^{D} together with the bridging nitrogen atom form a saturated, mono- or bicyclic, 3-10 membered heterocyclic ring system, which may be at least mono-substituted by one or more substituents independently selected from the group consisting of C₁₋₆ alkyl group, a -CO-C₁₋₆-alkyl group, a -CO-O- C₁₋₆-alkyl group, a -CO-NH- C₁₋₆-alkyl group, a -CS-NH- C₁₋₆-alkyl group, an oxo group, a C₁₋₆-alkyl group substituted with one or more hydroxy groups, a C₁₋₆-alkylene-O-C₁₋₆-alkyl group and a ―CO-NH₂ group and/or which may contain at least one further heteroatom selected from the group consisting of nitrogen, oxygen and sulphur as a ring member, and
wherein R^{E}, R^{F}, identical or different, represent hydrogen or a C₁₋₆-alkyl group, or R^{E} and R^{F} together with the bridging nitrogen atom form a saturated, mono- or bicyclic, 3-10 membered heterocyclic ring system, which may be at least mono-substituted by one or more, identical or different C₁₋₆ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo[2,1-b]thiazolyl, triazolyl and pyrazolyl.

If one or more of the residues R⁴-R⁸ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl , wherein the C₁₋₄-alkyl may in each case be branched or unbranched, and a phenyl group, more preferably be selected from the group consisting of hydroxy, F, Cl, Br, methoxy, ethoxy, CF₃ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues R⁴―R⁸ represents or comprises a linear or branched alkylene group, said alkylene group may preferably be selected from the group consisting of ―methylene -(CH₂)-, ethylene -(CH₂-CH₂)-, n-propylene -(CH₂-CH₂-CH₂)- or isopropylene ―(-C(CH₃)₂)-.

In another preferred embodiment of the invention the CB1 Antagonist or Inverse Agonist used according to the invention is selected from a compound of general formula I given above, wherein
R¹ represents an optionally at least mono-substituted phenyl group,
R² represents an optionally at least mono-substituted phenyl group,
R³ represents a saturated or unsaturated, optionally at least mono-substituted, optionally at least one heteroatom as ring member containing cycloaliphatic group, which may be condensed with an optionally at least mono-substituted mono- or polycyclic ring system, or R³ represents an optionally at least mono-substituted aryl or heteroaryl group, which may be condensed with an optionally at least mono-substituted mono- or polycyclic ring system, or R³ represents an ―NR⁴R⁵-moiety,
R⁴ and R⁵, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R⁶-moiety, or an -NR⁷R⁸-moiety,
R⁶ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R⁷ and R⁸, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
whereby preferably the following provisos (disclaimers) apply:
that R⁴ and R⁵ do not both represent a hydrogen atom, and that if one of the residues R⁴ and R⁵ represents a hydrogen atom or a linear or branched, saturated or unsaturated, substituted or unsubstituted aliphatic group, the other one of these residues R⁴ and R⁵ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an ―NH-phenyl-moiety, wherein the phenyl group may be at least mono-substituted, an unsubstituted or substituted thiazole group, or an unsubstituted or substituted [1,3,4]thiadiazole group.

Preferred is also the use of substituted pyrazoline compounds of general formula I given above, wherein R¹ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and R" for each substituent independently represent linear or branched C₁₋₆ alkyl, preferably R¹ represents a phenyl group, which is optionally substituted by one or more substituents selected from the group consisting of methyl, ethyl, F, Cl, Br and CF₃, more preferably R¹ represents a phenyl group, which is substituted with a chlorine atom in the 4-position, and R²-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred is the use of substituted pyrazoline compounds of general formula I given above, wherein R² represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R² represents a phenyl group, which is optionally substituted by one or more substituents selected from the group consisting of methyl, ethyl, F, Cl, Br and CF₃, more preferably R² a phenyl group, which is di-substituted with two chlorine atoms in the 2- and 4-position, and R¹ and R³-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to the use of substituted pyrazoline compounds of general formula I given above, wherein R³ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or R³ represents an optionally at least mono-substituted, 5- or 6-membered aryl or heteroaryl group, which may be condensed with an optionally at least mono-substituted mono- or polycyclic ring system, or R³ represents an -NR⁴R⁵-moiety, preferably R³ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or R³ represents an ―NR⁴R⁵-moiety, more preferably R³ represents a pyrrolidinyl group, a piperidinyl group or a piperazinyl group, whereby each of these groups may be substituted with one or more C₁₋₆-alkyl groups, or R³ represents an ―NR⁴R⁵-moiety and R¹, R² and R⁴-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore, the use of substituted pyrazoline compounds of general formula I given above are preferred, wherein R⁴ and R⁵, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an ―SO₂-R⁶-moiety, or an -NR⁷R⁸-moiety, preferably one of these residues R⁴ and R⁵ represents a hydrogen atom and the other one of these residues R⁴ and R⁵ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an ―SO₂-R⁶-moiety, or an -NR⁷R⁸-moiety, or R⁴ and R⁵, identical or different, each represent a C₁₋₆ alkyl group, more preferably one of these residues R⁴ and R⁵ represents a hydrogen atom and the other one of these residues R⁴ and R⁵ represents an optionally at least mono-substituted pyrrolidinyl group, an optionally at least mono-substituted piperidinyl group, an optionally at least mono-substituted piperazinyl group, an optionally at least mono-substituted triazolyl group, an ―SO₂-R⁶-moiety, or an -NR⁷R⁸-moiety, or R⁴ and R⁵, identical or different, represent a methyl group, an ethyl group, an n-propyl group, an isopropyl group, an n-butyl group, a sec-butyl group or a tert.-butyl group, and R¹-R³ and R⁶-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred is the use of substituted pyrazoline compounds of general formula I given above, wherein R⁶ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R⁶ represents a C₁₋₆-alkyl group, a saturated, optionally at least mono-substituted cycloaliphatic group, which may be condensed with a mono- or polycyclic ring-system, or a phenyl group, which is optionally substituted with one or more C₁₋₆ alkyl groups, and R¹-R⁵, R⁷and R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Moreover the use of substituted pyrazoline compounds of general formula I given above is preferred, wherein R⁷ and R⁸, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably represent a hydrogen atom or a C₁₋₆ alkyl radical, and R¹-R⁶ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferred is the use of compounds of general formula I given below, 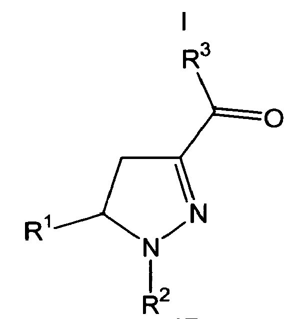 wherein
R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group, a piperidinyl group, a piperazinyl group, a homo-piperazinyl group, a morpholinyl group, or an -NR⁴R⁵-moiety,
R⁴ represents a hydrogen atom or a linear or branched C₁₋₆-alkyl group,
R⁵ represents a linear or branched C₁₋₆ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, or an -SO₂-R⁶-moiety, and
R⁶ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred is the use of substituted pyrazoline compounds selected from the group consisting of:
N-piperidinyl-5-(4-chloro-phenyl)-1-(2,4-dichlorophenyl)-4,5-dihydro-1H-pyrazole-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsufonamide,
optionally in the form of a corresponding N-oxide, or a corresponding salt, or a corresponding solvate.

In another aspect the present invention also provides a process for the preparation of substituted pyrazoline compounds of general formula I given above, according to which at least one benzaldehyde compound of general formula II 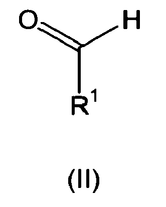 wherein R¹ has the meaning given above, is reacted with a pyruvate compound of general formula (III) 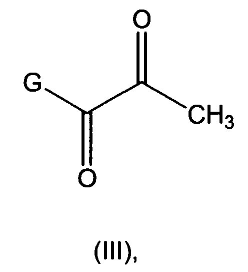 wherein G represents an OR group with R being a branched or unbranched C₁₋₆ alkyl radical, preferably an ethyl radical, or G represents an O⁻K group with K being a cation, preferably a monovalent cation, more preferably an alkali metal cation, even more preferably a sodium cation, to yield a compound of general formula (IV) 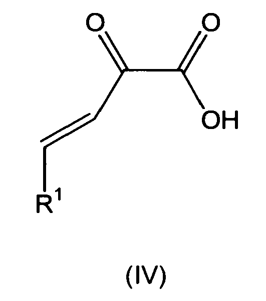 wherein R¹ has the meaning given above, which is optionally isolated and/or optionally purified, and which is reacted with an optionally substituted phenyl hydrazine of general formula (V) 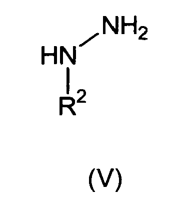 or a corresponding salt thereof, wherein R² has the meaning given above, under an inert atmosphere, to yield a compound of general formula (VI) 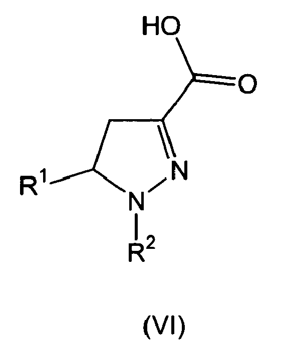 wherein R¹ and R² have the meaning as given above, which is optionally isolated and/or optionally purified, and optionally transferred under inert atmosphere to a compound of general formula (VII) 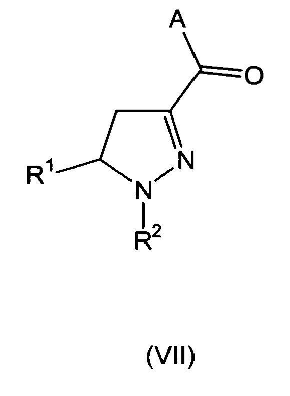 wherein the substituents R¹ and R² have the meaning given above and A represents a leaving group, via the reaction with an activating agent, said compound being optionally isolated and/or optionally purified, and at least one compound of general formula (VI) is reacted with a compound of general formula R³H, wherein R³ represents an ―NR⁴R⁵-moiety, wherein R⁴ and R⁵ have the meaning given above, to yield a substituted pyrazoline compound of general formula I, wherein R³ represents an ―NR⁴R⁵-moiety,
and/or at least one compound of general formula (VII) is reacted with a compound of the general formula R³H, in which R³ has the meaning given above to yield a compound of general formula (I) given above, which is optionally isolated and/or optionally purified.

The inventive process is also illustrated in scheme I given below:

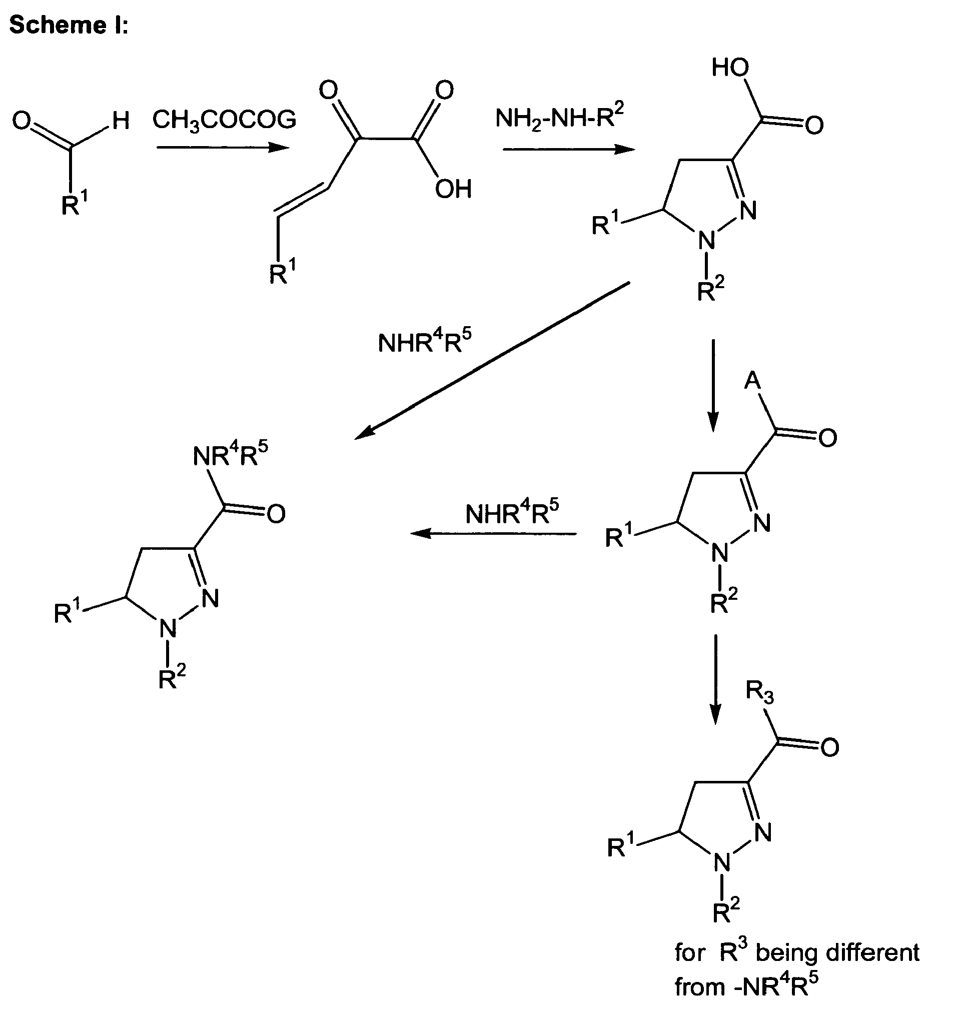

The reaction of the benzaldehyde compound of general formula II with a pyruvate compound of general formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a C₁₋₄ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula II with a pyruvate compound of general formula III is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (IV) with an optionally substituted phenyl hydrazin of general formula (V) is preferably carried out in a suitable reaction medium such as C₁₋₄-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VI) may be activated for further reactions by the introduction of a suitable leaving group according to conventional methods well known to those skilled in the art. Preferably the compounds of general formula (VI) are transferred into an acid chloride, an acid anhydride, a mixed anhydride, a C₁₋₄ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VII) is a mixed anhydride, said anhydride may preferably be prepared, for example, by reaction of the corresponding acid of general formula (VI) with ethyl chloroformiate in the presence of a base such as triethylamine or pyridine, in a suitable solvent.

The reaction of general formula (VII) with a compound of general formula HR³ to yield compounds of general general I, wherein R³ represents an -NR⁴R⁵ moiety is preferably carried out in presence of a base such as triethylamine in a reaction medium such as methylenchloride. The temperature is preferably in the range from 0°C to the boiling point of the reaction medium. The reaction time may vary over a broad range, e.g. from several hours to several days.

The reaction of general formula (VII) with a compound of general formula HR³ to yield compounds of general formula I, wherein R³ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341 (7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of FeCl₃, ZnCl₂ and AlCl₃, in a suitable reaction medium such as toluene, benzene, tetrahydrofurane or similar. The temperature is preferably in teh range from 0°C to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

In a further aspect the present invention also provides a process for the preparation of salts of substituted pyrazoline compounds of general formula (I) and stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I) or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The purification and isolation of the inventive substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The substituted pyrazoline compounds of general formula (I) given below, their stereoisomers, corresponding N-oxides, corresponding salts thereof and corresponding solvates are toxicologically acceptable and are therefore suitable as pharmaceutical active substances for the preparation of medicaments.

In another preferred embodiment of the invention the CB1 Antagonist or Inverse Agonist used according to the invention is selected from a compound of general formula II, 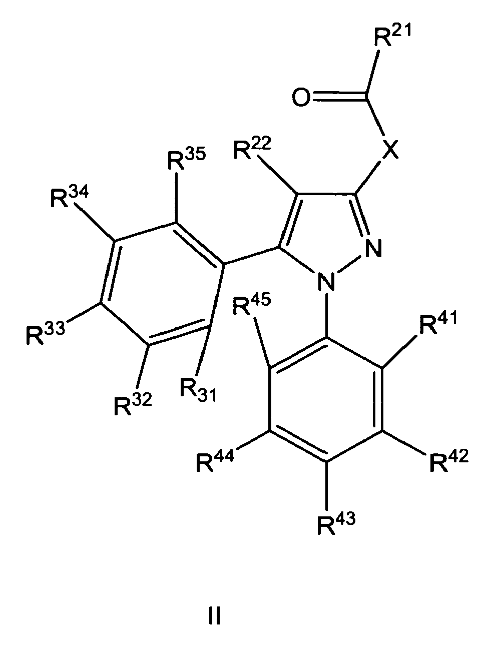 wherein
X is either a direct bond or a group (-CH₂)ₘ-N(R²³) in which R²³ is hydrogen or a (C₁-C₃)-alkyl and m is 0 or 1; and
R²¹ is
a group ―NR²⁴R²⁵ in which R²⁴ and R²⁵ are independently a (C₁-C₆)-alkyl; an optionally substituted non-aromatic (C₃-C₁₅) carbocyclic radical; an amino (C₁-C₄) alkyl group in which the amino is optionally disubstituted by a (C₁-C₃)-alkyl; a cycloalkyl-(C₁-C₃) alkyl in which the cycloalkyl is C₃-C₁₂; a phenyl which is unsubstituted or monosubstituted or polysubstituted by a halogen, by a (C₁-C₅)-alkyl or by a (C₁-C₅)-alkoxy; a phenyl (C₁-C₃)-alkyl; a diphenyl-(C₁-C₃)-alkyl; a naphthyl; an anthracenyl; a saturated 5- to 8-membered heterocyclic radical which is unsubstituted or substituted by a (C₁-C₃)-alkyl, by a hydroxyl or by a benzyl group; a 1-adamantylmethyl; an aromatic heterocycle unsubstituted, mono- or polysubstituted by a halogen, a (C₁-C₅)-alkyl, a (C₁-C₅)-alkoxy; a (C₁-C₃)-alkyl substituted by an aromatic heterocycle unsubstituted or mono- or polysubstituted by a halogen, a (C₁-C₅)-alkyl, a (C₁-C₅)-alkoxy, or else R²⁴ is hydrogen and R²⁵ is as defined above, or else R²⁴ and R²⁵ together with the nitrogen atom to which they are bonded, form a saturated 5-to 8-membered heterocyclic radical, said heterocyclic radical being other than morpholine when R³¹, R³², R³³, R³⁴ and R³⁵ and R⁴¹, R⁴², R⁴³, R⁴⁴ and R⁴⁵ are all hydrogen;
a group R²⁵ as defined above, when X is ―(CH₂)ₘN(R²³)-; or
a group R²⁶, when X is a direct bond, R²⁶ being a (C₁-C₃)-alkyl; a (C₃-C₁₂)-cycloalkyl which is unsubstituted or substituted by a (C₁-C₅)-alkyl: a phenyl-(C₁-C₃)-alkyl which is unsubstituted or substituted by a halogen or by a (C₁-C₅)-alkyl; a cycloalkyl-(C₁-C₃)-alkyl in which the cycloalkyl is C₃-C₁₂ and is unsubstituted or substituted by a (C₁-C₅)-alkyl; or a 2-norbomylmethyl;
R³¹, R³², R³³, R³⁴ and R³⁵ and R⁴¹, R⁴², R⁴³, R⁴⁴ and R⁴⁵ are identical or different and are independently hydrogen, a chlorine, Iodine or bromine atom, a (C₁-C₃)-alkyl, a (C₁-C₃)-alkoxy, a trifluormethyl, or a nitro group and R³³ is optionally a phenyl group,
R²² is hydrogen or a (C₁-C₃)-alkyl;
one of its salts.
One known Compound falling under this general formula II is Rimonabant (SR 141716) known from many patents and applications (e.g. EP576357 B1, EP 656354 B1 and US 5,624,941) included here by reference.

In another preferred embodiment of the invention the CB1 Antagonist or Inverse Agonist used according to the invention is selected from a compound of general formula II 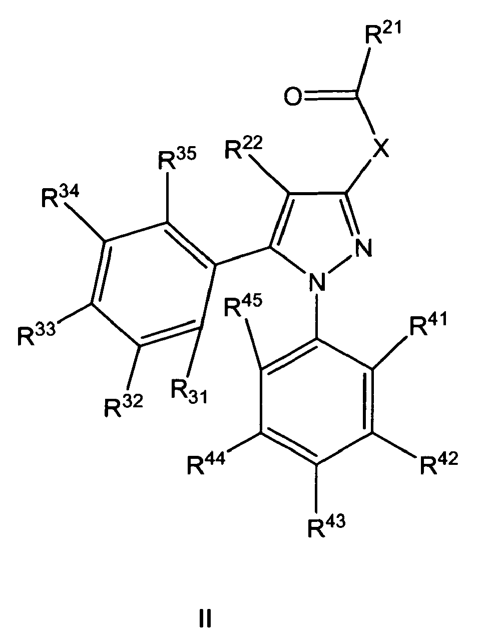 wherein
X is a direct bond or a group (-CH₂)ₘ-N(R²³) in which R²³ is hydrogen or a (C₁-C₃)-alkyl and m is 0 or 1; and
R²¹ is selected from
― NR²⁴R²⁵ in which R²⁴ is hydrogen and R²⁵ is phenyl, (C₁-C₈)-alkyl, or 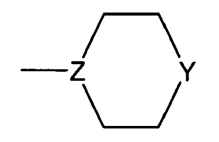 with Z being N or CH and Y being O, NR²⁷ or CHR²⁷, with R²⁷ being hydrogen or hydroxyl;
R³¹, R³², R³³, R³⁴ and R³⁵ and R⁴¹, R⁴², R⁴³, R⁴⁴ and R⁴⁵ are identical or different and are independently hydrogen, a halogen atom, a (C₁-C₈)-alkyl, a (C₁-C₃)-alkoxy, a trifluormethyl, or a nitro group and R³³ is optionally a phenyl group,
R²² being selected from hydrogen, CN or a (C₁-C₃)-alkyl; optionally in the form of a corresponding N-oxide, a corresponding salt or a corresponding solvate.

In another preferred embodiment of the invention the CB1 Antagonist or Inverse Agonist used according to the invention is selected from a compound of general formula II in which R³¹, R³², R³⁴, R³⁵, R⁴², R⁴⁴ and R⁴⁵ are hydrogen.

In another preferred embodiment of the invention the CB1 Antagonist or Inverse Agonist used according to the invention is selected from a compound of general formula II in which R³³, R⁴¹and R⁴³ are selected from hydrogen, iodine, chlorine, bromine, (C₁-C₈)-alkyl or (C₁-C₃)-alkoxy.

In another preferred embodiment of the invention the CB1 Antagonist or Inverse Agonist used according to the invention is selected from a compound of general formula II is selected from:
- 1-(2,4-dichlorophenyl)-5-(4-chlorophenyl)-4-methyl-N-(piperidin-1-yl)-1 H-pyrazole-3-carboxamide;
- 1-(2,4-dichlorophenyl)-5-(4-bromophenyl)-4-methyl-N-(piperidin-1-yl)-1H-pyrazole-3-carboxamide;
- 1-(2,4-dichlorophenyl)-5-(4-chlorophenyl)-4-ethyl-N-(piperidin-1-yl)-1H-pyrazole-3-carboxamide;
- 1-(2,4-dichlorophenyl)-5-(4-bromophenyl)-4-ethyl-N-(piperidin-1-yl)-1H-pyrazole-3-carboxamide;
- 1-(2,4-dichlorophenyl)-5-(4-chlorophenyl)-N-(piperidin-1-yl)-1H-pyrazole-3-carboxamide;
- 1-(2,4-dichlorophenyl)-5-(4-bromophenyl)-N-(piperidin-1-yl)-1H-pyrazole-3-carboxamide or
- 1-(2,4-dichlorophenyl)-5-(4-iodophenyl)-4-methyl-N-(pipeddin-1-yl)-1H-pyrazole-3-carboxamide; or
- 1-(2,4-dichlorophenyl)-5-(4-chlorophenyl)-N-(4-hyd roxypiperidin-1-yl)-4-methyl-1 H-pyrazole-3-carboxamide optionally in the form of a corresponding N-oxide, a corresponding salt or a corresponding solvate.

In another preferred embodiment of the invention the CB1 Antagonist or Inverse Agonist used according to the invention is selected from a compound, optionally in the form of a corresponding N-oxide, a corresponding salt or a corresponding solvate; identified through the Reference-Table (see Annex 1 and 2).

**Reference Table:**

| **Literature (included by reference and included as pages** | **Page: in application (in article)** | **Figure No.: (Group No.)** | **Structure No.** | **Structure Code/Name** |
|---|---|---|---|---|
| **ANNEX 1:** | 73 (499) | 1 (I) | 1 | Rimonabant |
| Lange and Kruse, "Recent advances in CB1 cannabinoid receptor antagonists", Current Opinion in Drug Discovery & Development, 2004, 7(4): 498-506 | | | 2 | |
| | | | 3 | |
| | | | 4 | CP-272871 |
| | | | 5 | NIDA-41020 |
| | | | 6 | |
| | | | 7 | |
| | 74 (500) | 2 (II) | 8 | |
| | | | 9 | |
| | | | 10 | |
| | | | 11 | |
| | | | 12 | |
| | 75 (501) | 3 (III) | 13 | |
| | | | 14 | |
| | | | 15 | |
| | | | 16 | |
| | | | 17 | |
| | 76 (502) | 4 (IV) | 18 | |
| | | | 19 | |
| | | | 20 | |
| | | | 22 | |
| | | 5 (V) | 23 | SLV 319 |
| | | | 24 | SLV 326 |
| | | | 25 | |
| | | | 26 | |
| | | | 27 | |
| | 77 (503) | 6 (VI) | 28 | |
| | | | 29 | |
| | | | 30 | |
| | | | 31 | |
| | | | 32 | |
| | | 7 (VII) | 33 | |
| | | | 34 | |
| | | | 35 | |
| | 78 (504) | 8 (VIII) | 36 | O-1918 |
| **ANNEX 2:** | 85 (57) | 1 (IX) | 1 | Rimonabant |
| Smith and Fathi, "Recent advances in the research and development of CB1 antagonists", IDrugs 2005 8(1):53-66 | | | 2 | O-889 |
| | | | 3 | SR-147778 |
| | | | 4 | |
| | | | 6 | |
| | | | 7 | |
| | | 2 (X) | 8 | SLV-319 |
| | | | 9 | |
| | | | 10 | |
| | 86 (58) | 3 (XI) | 11 | |
| | | | 12 | |
| | | | 13 | |
| | | | 14 | |
| | | | 15 | |
| | | | 16 | |
| | | | 17 | |
| | | | 18 | |
| | 87 (59) | 4 (XII) | 19 | |
| | | | 20 | |
| | | | 21 | |
| | | | 22 | |
| | | | 23 | |
| | | | 24 | |
| | | | 25 | |
| | | 5 (XIII) | 26 | |
| | | | 27 | |
| | | | 28 | |
| | | | 29 | |
| | | 6 (XIV) | 30 | |
| | | | 31 | |
| | 88 (60) | 7 (XV) | 32 | |
| | | | 33 | |
| | | | 34 | |
| | | 8 (XVI | 35 | |
| | | | 36 | |
| | | | 37 | |
| | | | 38 | |
| | | | 39 | |
| | | 9 (XVII) | 40 | |
| | | | 41 | |
| | | | 42 | |
| | (89) 61 | 10 (XVIII) | 43 | |
| | | | 44 | |
| | | | 45 | |
| | | | 46 | |
| | | | 47 | |
| | | | 48 | |
| | | | 49 | |
| | | | 50 | |
| | | 11 (XIX) | 51 | |
| | | | 52 | |
| | | | 53 | |
| | | | 54 | |
| | | | 55 | |
| | | | 56 | |
| | | | 57 | |
| | | | 58 | |
| | (90) 62 | 12 (XX) | 59 | |
| | | | 60 | |
| | | | 61 | |
| | | | 62 | |
| | | | 63 | |

The Literature (identified as ANNEX 1 and ANNEX 2 below) mentioned in the Reference Table is included in this description by reference completely but also added as ANNEXES on pages: 72 to 80 and 81 to 94 respectively of this application.
**ANNEX 1:**
   Lange and Kruse, "Recent advances in CB1 cannabinoid receptor antagonists", Current Opinion in Drug Discovery & Development, 2004, 7(4): 498-506
**ANNEX 2:**
   Smith and Fathi, "Recent advances in the research and development of CB1 antagonists", IDrugs 2005 8(1):53-66

In another preferred embodiment of the invention the CB1 Antagonist or Inverse Agonist used according to the invention is selected from the group of:
- 1,5-Diarylpyrazole cpds., preferably from close analogs to Group (I) according to the reference table (above);
- tricyclic pyrazole cpds., preferably from close analogs to Group (II) according to the reference table (above);
- tetracyclic pyrazole cpds., preferably from close analogs to Group (II) according to the reference table (above);
- five-membered aromatic heterocyclic ring bioisostere cpds., preferably from close analogs to Group (III) according to the reference table (above);
- six-membered aromatic heterocycle cpds., preferably from close analogs to Group (IV) according to the reference table (above);
- 3,4-diarylpirazoline cpds., preferably from close analogs to Group (V) according to the reference table (above);
- substituted amide cpds., preferably from close analogs to Group (VI) according to the reference table (above);
- benzodioxole derivative cpds., preferably from close analogs to Group (VII) according to the reference table (above);
- biphenyl derivative cpds., preferably from close analogs to Group (VIII) according to the reference table (above);
- 1,5-Diaryl-pyrazole cpds., preferably from close analogs to Group (IX) according to the reference table (above);
- 3,4-Diaryl-4,5-dihydropyrazole cpds., preferably from close analogs to Group (X) according to the reference table (above);
- 4,5-Diaryl-imidazole cpds., preferably from close analogs to Group (XI) according to the reference table (above);
- 1,2-Diaryl-imidazole cpds., preferably from close analogs to Group (XI) according to the reference table (above);
- 2,3-Diaryl-1,2,4-triazole cpds., preferably from close analogs to Group (XII) according to the reference table (above);
- Pyrrole cpds., preferably from close analogs to Group (XIII) according to the reference table (above);
- Thiazole cpds., preferably from close analogs to Group (XIV) according to the reference table (above);
- lmidazolidinedione cpds., preferably from close analogs to Group (XV) according to the reference table (above);
- Six-membered-ring-core cpds., preferably from close analogs to Group (XVI) according to the reference table (above);
- Four-membered-ring-core cpds., preferably from close analogs to Group (XVII) according to the reference table (above):

- Bicyclic-core cpds., preferably from close analogs to Group (XVIII) according to the reference table (above);
- Constrained tricyclic analog cpds., preferably from close analogs to Group (XIX) according to the reference table (above);
- Constrained tetracyclic analog cpds., preferably from close analogs to Group (XIX) according to the reference table (above); or
- Acyclic analog cpds., preferably from close analogs to Group (XX) according to the reference table (above);
optionally in the form of a corresponding N-oxide, a corresponding salt or a corresponding solvate.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.
The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

### Pharmacological Methods

### I. In-vitro determination of affinity to CB1/CB2-Receptors

In-vitro determination of the affinity of the Canabinoid Receptor Compounds used according to the invention to CB₁/CB₂-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human CB₁ and CB₂ receptors of Receptor Biology, Inc. are used. The radioligand used for both receptors is [³H]-CP55940. The respective parts of the description are hereby incorporated by reference and forms part of the present disclosure.

### II. In-vitro bioassay system for determination of anti-inflammatory or immunomodulatory activity

An inflammation process involves a variety of cell types including those of the immune system, including leukocytes and macrophages. This results in a complex program of gene expression that culminates in cell proliferation and acquisition of effectors functions. Coordinated induction of genes involved in the inflammatory process are usually regulated at the level of transcription, being dependent on the activation of transcription factors such as nuclear factor of activated T cells (NF-AT) and nuclear factor NF-kB.

In order to determine the effects of CB1 Antagonists or inverse Agonists on the modulation of the activation of the immune system and inflammation, proliferation and transcriptional assays have been performed in the human leukemia T cell line Jurkat and in the human monocytic THP-1 cell line.

As a consequence drugs able to interfere with transcriptional activation of genes involved in the inflammatory process and in the immune response could be considered as potential anti-inflammatory and/or immunomodulatory agents.

### Examples:

### Example 1:

### N-piperidinyl-5-(4-chlorophenyl)-1-(2,4-dichlorophenyl)-4,5-dihydro-1H-pyrazole-3-carboxamide

### a) 4-(4-chlorophenyl)-2-oxo-3-butenoic acid

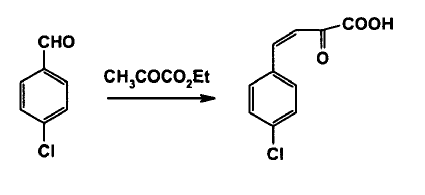

In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, cm⁻¹) : 3500-2500, 1719,3, 1686,5, 1603,4, 1587,8, 1081,9.
¹H NMR(CDCl₃, δ) : 7,4 (d, J=8,4Hz, 2H), 7,5 (d, J=16,1 Hz, 1 H), 7,6 (d, J=8,4Hz, 2H), 8,1(d, J=16,1Hz, 1H).

### b) 5-(4-chlorophenyl)-1-(2,4-dichlorophenyl)-4,5-dihydro-pyrazole-3-carboxylic acid

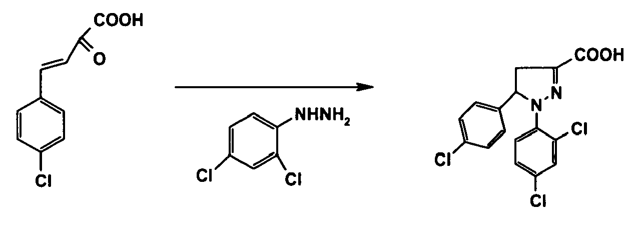

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, cm⁻¹) : 3200-2200, 1668,4, 1458, 1251,4, 1104,8.
¹H NMR (CDCl₃, δ) : 3,3 (dd, 1 H), 3,7 (dd, 1 H), 5,9 (dd, 1 H), 7,09-7,25 (m, 7H).

### (c) 5-(4-chlorophenyl)-1-(2,4-dichlorophenyl)-4,5-dihydro-pyrazole-3-carboxylic acid chloride

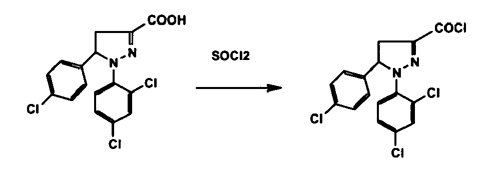

Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, cm-¹): 1732,3, 1700, 1533,3, 1478,1, 1212,9, 826,6.

### d) N-piperidinyl-5-(4-chlorophenyl)-1-(2,4-dichlorophenyl)-4,5-dihydropyrazole-3-carboxamide [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

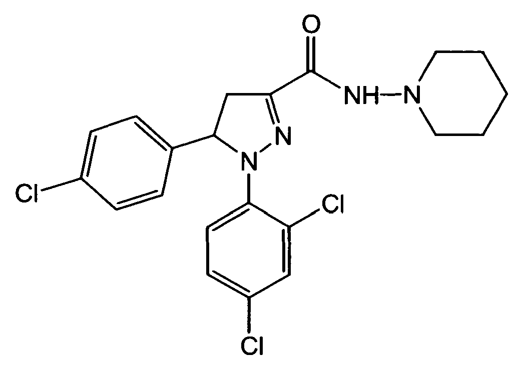

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.
IR (KBr, cm⁻¹) : 3222,9, 2934,9, 1647,4, 1474,7, 1268,3, 815,6.
¹H NMR (CDCl₃, δ) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1 H).

The compounds according to the following examples 2-6 have been prepared analogously to the process described in Example 1.

### Example 2:

### 5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 °C.
IR (KBr, cm⁻¹): 3448, 1686, 1477, 1243, 1091, 821.
¹H NMR(CDCl₃, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

### Example 3:

### 5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155°C.
IR (KBr, cm⁻¹) : 3433, 1685, 1477, 1296, 1246, 1088, 1014, 825.
¹H NMR (CDCl₃, δ): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

### Example 4:

### 5-(4-Chloro-phenyl)-1-(2,4-dichloro-phenyl)-4,5-dihydro-1H-pyrazole-3-carboxylic acid diethylamide

This compound was obtained in form of an oil.
IR (film, cm⁻¹) : 2974, 1621, 1471, 1274, 1092, 820.
¹H NMR (CDCl₃, δ): 1,2 (m, 6H), 3,3-3,9 (m, 6H), 5,6 (dd, J=5,8 and 11,7 Hz, 1 H), 7-7,25 (m, 7H).

### Example 5:

### [5-(4-Chloro-phenyl)-1-(2,4-dichloro-phenyl)-4,5-dihydro-1H-pyrazol-3-yl]-piperidin-1-yl-methanone

Melting point: 105-110°C.
IR (KBr, cm⁻¹): 2934, 1622, 1470, 1446, 1266, 1010, 817.
¹H NMR (CDCl₃, δ): 1,7 (m, 6H), 3,4 (dd, J=5,7 and 17,9Hz, 1 H), 3,7 (m, 3H), 3,9 (m, 2H), 5,6 (dd, J=6,1 y 11,9 Hz, 1 H), 7-7,25 (m, 7H).

### Example 6:

### N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, cm⁻¹) : 1697, 1481, 1436, 1340, 1169, 1074, 853.
¹H NMR (CDCl₃, δ): 2,4 (s, 3H), 3,2 (dd, J=6,6 and 18,3Hz, 1 H), 3,6 (dd, J=12,8 and 18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1H).

### Example 7:

### N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.
IR (KBr, cm⁻¹): 3202, 1678, 1654, 1474, 1309, 1107.
¹H-NMR (CDCl₃, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1 H.)

### Pharmacological Data:

### I. In-vitro determination of affinity to CB₁/CB₂-Rezeptors

The affinity of the inventive substituted pyrazoline compounds to CB₁/CB₂ receptors was determined as described above. Some of the values obtained are given in the following table I:

**Table I:**

| Compound according to Example | CB₁-Receptor Radiologand:[³H]-CP55940 | | CB₂-Receptor Radiologand:[³H]-CP55940 | |
|---|---|---|---|---|
| | % Inhibition 10⁻⁶ M | Kᵢ(nM) | % Inhibition 10⁻⁶ M | Kᵢ(nM) |
| 1 | 93% | < 25 | 33% | > 1000 |
| 5 | 79% | 111 | 54% | ≈ 1000 |

As can be seen from the values given in table 1 the pyrazoline compounds according to general formula I are particularly suitable for regulating the CB₁-Receptor.

### II. In-vivo bioassay system for determination of cannabinoid activity

The determinination of cannabinoid activity in-vivo was determined as described above. Some of the values obtained are given in the following table II:

**Table II:**

| Compound according to example: | dosis administered: 5 mg/kg i.v. | | | | dosis administered 5 mg/kg i.v. prior to Win 55212-2 in a dose of 1,25mg/kg i.v. | | | |
|---|---|---|---|---|---|---|---|---|
| | Agonistic effect | | | | Antagonistic Effect | | | |
| | A | B | C. | D | A | B. | C | D |
| 1 | 0 | 0 | 0 | 0 | 74 | 100 | 100 | 100 |
| 5 | 0 | 50 | 0 | 0 | 50 | 40 | 20 | 20 |

| | | | | | | | | |
|---|---|---|---|---|---|---|---|---|
| i.v. intravenous A: Hot-Plate test B: Hypothermia C: Catalepsy D: Sedation | | | | | | | | |

As can be seen from the values given in table II the pyrazoline compounds according to general formula I show an antagonistic effect. Especially the Compound according to example 1 is a CB1 antagonist.

### III. In-vitro testing for immunmodulatory or antoinflammatory activity

### 1. Effects of the compound according to example 1 on cell viability

The viability of the cells was measured by the use of the tetrazolium salt WST-1 (Roche Molecular Biochemicals). Unstimulated Jurkat cells or LPS (5 µg/ml) treated THP-1 cells were incubated for 24 or 18 hours respectively in the presence of increasing doses of the compound according to example 1 (1, 10, 100, 1000 and 10000 nM) and incubated with 10% of WST-1 reagent for 1 hour. Quantification of the formazan dye produced by metabolically active cells was spectrophotometrically measured at 450 nm and a reference wavelength of 630 nm. Data are represented as percentage of viability considering 100% the value of absorbance obtained in the absence of example 1 (Table 1).

**Table 1.**

| | **0** | **10 nM** | **100 nM** | **1000 nM** | **10000 nM** |
|---|---|---|---|---|---|
| **Jurkat (Cont)** | 100.0 % | 92.6 % | 95.4 % | 96.3 % | 97.7 % |
| **THP-1 (LPS)** | 100.0 % | 105.1 % | N.D. | 110.2 % | 80.2 % |

### 2. Inhibition of NFAT and NF-κB-mediated transcriptional activation

It was also analyzed how these compounds affect transcriptional induction mediated by the transcription factors Nuclear factor of activated T cells (NFAT) or Nuclear factor (NF)-κB. In these experiments, Jurkat T cells or THP-1 cells were transiently transfected by the Lipofectamine2000 reagent (Invitrogen), with luciferase reporter constructs whose expression are directed by three copies of the Interleukin-2 (IL-2) NFAT responsive element (NFAT-LUC) or by three copies of an NF-κB responsive element (NF-κB-LUC). Cells were treated with increased doses of the compound according to example 1 in the presence or absence of stimulation with the phorbol ester PMA (15 ng/ml) plus the A23187 calcium ionophore (1µM) for 6 hours in Jurkat cells or with LPS (5 µg/ml) for 18 hours in THP-1 cells and luciferase activity was measured in cell lysates. Results obtained are represented as percentage of activation, considering 100% the value of luciferase activity obtained in stimulated cells in the absence of the compound according to example 1. The approximate IC-50 (µM) values for the inhibition of the transcriptional activation of NFAT and NF-κB are also shown (Table 2).

**Table 2.**

| | **NFAT-LUC** | **NF-κB -LUC** | **NF-κB-LUC** |
|---|---|---|---|
| | **Jurkat (PMA+lon)** | **Jurkat (PMA+lon)** | **THP-1 (LPS)** |
| **0** | 100.0% | 100.0% | 100.0% |
| **0.01 µM** | 72.4 % | 94.7 | 100.4 % |
| **1 µM** | 56.8% | 74.4 % | 104.6 % |
| **10 µM** | 7.4% | 39.3% | 3.4% |
| | | | |
| **IC-50 (µM)** | 3 | 7 | 6 |

The same experiment was performed with SR141716, known as Rimonabant.

| | | | |
|---|---|---|---|
| **IC-50 (µM)** | 0.2 | 1 | 5 |

### 3. Inhibition of transcriptional induction of gene expression

The compound according to example 1 was measured for its ability to regulate gene transcription in immune activation through the modulation of the transcriptional activity of luciferase reporter constructs whose expression is directed by the promoter region of genes involved in the inflammation process and immune activation, such as cyclooxygenase-2 (COX-LUC), tumor necrosis factor α (TNF-LUC), Interleukin-2 (IL-2-LUC), Interleukin-6 (IL-6-LUC) and the inducible Nitric Oxide Synthase (iNOS-LUC). Jurkat T cells or THP-1 cells were transiently transfected with these constructs as described above, and treated with different doses of The compound according to example 1 in the presence or absence of stimulation with PMA plus Ion (Jurkat) or LPS in a similar way as for NFAT-LUC and NFKB-LUC reporter analysis. Results are represented as percentage of activation, considering 100% the value of luciferase activity obtained in stimulated cells in the absence of the compound according to example 1. Approximated IC50 (µM) values obtained for inhibition of the transcriptional activation are summarized in tables 3 (Jurkat) and 4 (THP-1).

**Table 3.**

| | **COX2-LUC** | **TNF-LUC** | **IL-2-LUC** | **IL-6-LUC** |
|---|---|---|---|---|
| | **Jurkat (PMA+lon)** | **Jurkat (PMA+lon)** | **Jurkat (PMA+lon)** | **Jurkat (PMA+lon)** |
| **0** | 100.0 % | 100.0 % | 100.0 % | 100.0 % |
| **0.01 µM** | 102.7 % | 59.5 % | 85.9% | 82.3% |
| **1µM** | 93.2 % | 29.9% | 49.0 % | 61.8% |
| **10 µM** | 31.5% | 13.7% | 9.9% | 18.6% |
| | | | | |
| **IC-50 (µM)** | 8 | 0.1 | 1 | 4 |

The same experiment was performed with SR141716, known as Rimonabant.

| | | | | |
|---|---|---|---|---|
| **IC-50 (µM)** SR141716 | >10 | 0.01 | 0.1 | 1 |

**Table 4.**

| | **COX2-LUC** | **TNF-LUC** | **iNOS-LUC** |
|---|---|---|---|
| | **THP-1 (LPS)** | **THP-1 (LPS)** | **THP-1 (LPS)** |
| **0** | 100.0 % | 100.0 % | 100.0 % |
| **0.01 µM** | 102.7 % | 104.4% | 89.5% |
| **1 µM** | 59.6 % | 65.4% | 71.3% |
| **10 µM** | 6.8% | 12.7% | 13.2% |
| | | | |
| **IC-50 (µM)** | 2 | 3 | 4 |

The same experiment was performed with SR141716, known as Rimonabant.

| | | | |
|---|---|---|---|
| **IC-50 (µM)** | 6 | 3 | 3 |

### 4. Inhibition of cytokine production

In addition to the transcriptional assays, we have also determined the effects of the compound according to example 1 in the secretion of TNFα and IL-2 to the culture medium. Jurkat T cells were treated with different doses of the compound according to example 1 in the presence or absence of stimulation with PMA plus Ion for 24 hours. Supematants were collected and cytokines were measured by specific ELISA for each cytokine (Bender MedSystems). Results are represented as percentage of cytokine production, considering 100% the maximal levels of cytokine production (pg/ml) obtained in PMA+lon stimulated Jurkat cells in the absence of the compound according to example 1. Approximated IC50 (µM) values obtained for inhibition of the cytokine production are summarized in table 5.

**Table 5.**

| | **TNF** | **IL-2** |
|---|---|---|
| | **Jurkat (PMA+lon)** | **Jurkat (PMA+lon)** |
| **0** | 100.0 % | 100.0 % |
| **0.01 µM** | 79.5 % | 97.5 % |
| **1 µM** | 80.3 % | 98.7 % |
| **10 µM** | 11.5% | 13.2% |
| | | |
| **IC-50 (µM)** | 5 | 6 |

The same experiment was performed with SR141716, known as Rimonabant

| | | |
|---|---|---|
| **IC-50 (µM)** | 6 | 7 |

In summary, it can be said that the compound according to example 1 as well as Rimonabant does display anti-inflammatory and immunomodulatory properties, interfering with the transcriptional activation of genes involved in inflammation and in the immune response such as cytokines (IL-2, IL-6 and TNFα), COX-2 and iNOS.

## Claims

1. Use of a CB1 antagonist or inverse agonist for the production of a medivament for the production of a medivament for the treatment and/or prophylaxis of an inflammation that involves expression of genes involved in inflammation and/or in the immune response.

2. Use according to claim 1, **characterized in that** the genes involved are selected from cytokines (IL-2, IL-6 and TNFα), COX-2 and iNOS.

3. Use of a CB1 antagonist or inverse agonist for the production of a medicament for the treatment and/or prophylaxis of inflammation based on an immunologic effect.

4. Use of a CB1 antagonist or inverse agonist for the production of a medicament for the treatment and/or prophylaxis of immunologic disorders that involve expression of genes involved in inflammation and/or in the immune response.

5. Use of a CB1 antagonist or inverse agonist for the production of a medicament for the treatment and/or prophylaxis of inflammatory heart diseases.

6. Use according to claim 5, **characterized in that** the inflammatory heart diseases involve expression of genes involved in inflammation and/or in the immune response.

7. Use according to claim 5, **characterized in that** the inflammatory heart disease is angina pectoris or atherosclerosis.

8. Use according to any of claim 1 to 7 in which the CB1-Antagonist is selected from a substituted pyrazoline compounds of general formula I, 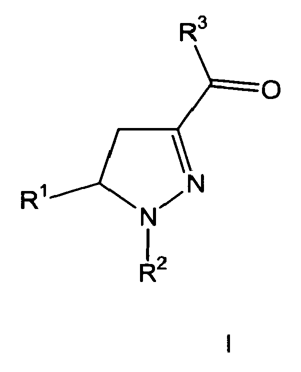 wherein
R¹ represents an optionally at least mono-substituted phenyl group;
R² represents an optionally at least mono-substituted phenyl group;
R³ represents a saturated or unsaturated, optionally at least mono-substituted, optionally at least one heteroatom as ring member containing cycloaliphatic group, which may be condensed with an optionally at least mono-substituted mono- or polycyclic ring system; or R³ represents an optionally at least mono-substituted aryl or heteroaryl group, which may be condensed with an optionally at least mono-substituted mono- or polycyclic ring system; or R³ represents an ―NR⁴R⁵-moiety,
R⁴ and R⁵, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an ―SO₂-R⁶-moiety; or an - NR⁷R⁸-moiety, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

9. Use according to claim 8, **characterized in that** in a compound according to formula I R¹ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R¹ represents a phenyl group, which is optionally substituted by one or more substituents selected from the group consisting of methyl, ethyl, F, Cl, Br and CF₃, more preferably R¹ represents a phenyl group, which is mono-substituted with a chlorine atom in the 4-position.

10. Use according to claim 8 or 9, **characterized in that** in a compound according to formula I R² represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and optionally R" for each substituent independently represent linear or branched C₁₋₆ alkyl, preferably R² represents a phenyl group, which is optionally substituted by one or more substituents independently selected from the group consisting of methyl, ethyl, F, Cl, Br and CF₃, more preferably R² represents a phenyl group, which is di-substituted with two chlorine atoms in its 2- and 4-position.

11. Use according to one or more of claims 8 to 10, **characterized in that** in a compound according to formula I R³ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or R³ represents an optionally at least mono-substituted, 5- or 6-membered aryl or heteroaryl group, which may be condensed with an optionally at least mono-substituted mono- or polycyclic ring system, or R³ represents an ―NR⁴R⁵-moiety, preferably R³ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or R³ represents an - NR⁴R⁵-moiety, more preferably R³ represents a pyrrolidinyl group, a piperidinyl group or a piperazinyl group, whereby each of these groups may be substituted with one or more C₁₋₆-alkyl groups, or R³ represents an -NR⁴R⁵-moiety.

12. Use according to one or more of claims 8-11, **characterized in that** in a compound according to formula I R⁴ and R⁵, identical or different, represent a hydrogen atom; an unbranched or branched, saturated or unsaturated, optionally at least mono-substituted C₁₋₆-aliphatic radical; a saturated or unsaturated, optionally at least mono-substituted, optionally at least one heteroatom as ring member containing C₃₋₈-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group; an -SO₂-R⁶-moiety; or an -NR⁷R⁸-moiety, preferably one of these residues R⁴ and R⁵ represents a hydrogen atom and the other one of these residues R⁴ and R⁵ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an ―SO₂-R⁶-moiety; or an -NR⁷R⁸-moiety, or R⁴ and R⁵, identical or different, each represent a C₁₋₆ alkyl group, more preferably one of these residues R⁴ and R⁵ represents a hydrogen atom and the other one of these residues R⁴ and R⁵ represents an optionally at least mono-substituted pyrrolidinyl group; an optionally at least mono-substituted piperidinyl group; an optionally at least mono-substituted piperazinyl group; an optionally at least mono-substituted triazolyl group; an ―SO₂-R⁶-moiety; or an - NR⁷R⁸-moiety, or R⁴ and R⁵, identical or different, represent a methyl group, an ethyl group, an n-propyl group, an isopropyl group, an n-butyl group, a sec-butyl group or a tert.-butyl group.

13. Use according to one or more of claims 8-12, **characterized in that** in a compound according to formula I R⁶ represents a linear or branched, saturated or unsaturated, optionally at least mono-substituted C₁₋₆ aliphatic group; a saturated or unsaturated, optionally at least mono-substituted, optionally at least one heteroatom as ring member containing C₃₋₈ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R⁶ represents a C₁₋₆-alkyl group; a saturated, optionally at least mono-substituted cycloaliphatic group, which may be condensed with a mono- or polycyclic ring-system; or a phenyl group, which is optionally substituted with one or more C₁₋₆ alkyl groups.

14. Use according to one or more of claims 8-13, **characterized in that** in a compound according to formula I R⁷ and R⁸, identical or different, represent a hydrogen atom; an unbranched or branched, saturated or unsaturated, optionally at least mono-substituted C₁₋₆ aliphatic radical; a saturated or unsaturated, optionally at least mono-substituted, optionally at least one heteroatom as ring member containing C₃₋₈ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably R⁷ and R⁸, identical or different, represent a hydrogen atom; or a C₁₋₆ alkyl radical.

15. Use according to one or more of claims 8 to 14, wherein in a compound according to formula I 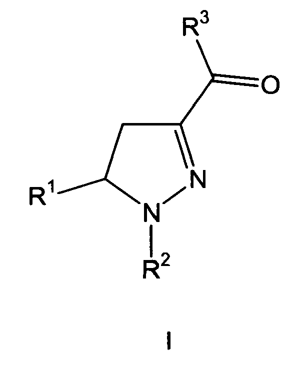 R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group; a piperidinyl group; a piperazinyl group; a homo-piperazinyl group; a morpholinyl group; or an ―NR⁴R⁵-moiety,
R⁴ represents a hydrogen atom or a linear or branched C₁₋₆-alkyl group,
R⁵ represents a linear or branched C₁₋₆ alkyl group; an SO₂-R⁶-moiety; a pyrrolidinyl group; a piperidinyl group; a piperazinyl group; a homo-piperazinyl group; a morpholinyl group; or a triazolyl group, whereby each of the heterocyclic rings may be substituted with one or more, identical or different, C₁₋₆-alkyl groups, and
R⁶ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

16. Use according to one or more of claims 8 to 15 wherein a compound according to formula I is selected from the group consisting of:
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

17. Use according to any claim 1 to 7 in which the CB1 Antagonist or Inverse Agonist is selected from a compound of general formula II, 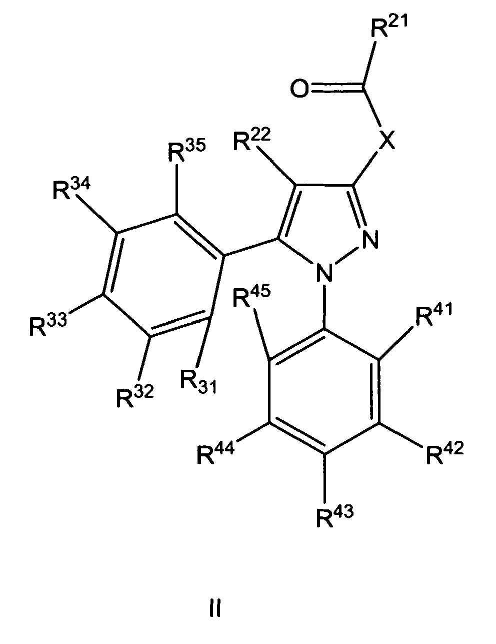 wherein
X is either a direct bond or a group (-CH₂)ₘ-N(R²³) in which R²³ is hydrogen or a (C₁-C₃)-alkyl and m is 0 or 1; and
R²¹ is
a group ―NR²⁴R²⁵ in which R²⁴ and R²⁵ are independently a (C₁-C₆)-alkyl; an optionally substituted non-aromatic (C₃-C₁₅) carbocyclic radical; an amino (C₁-C₄) alkyl group in which the amino is optionally disubstituted by a (C₁-C₃)-alkyl; a cycloalkyl-(C₁-C₃) alkyl in which the cycloalkyl is C₃-C₁₂; a phenyl which is unsubstituted or monosubstituted or polysubstituted by a halogen, by a (C₁-C₅)-alkyl or by a (C₁-C₅)-alkoxy; a phenyl (C₁-C₃)-alkyl; a diphenyl-(C₁-C₃)-alkyl; a naphthyl; an anthracenyl; a saturated 5- to 8-membered heterocyclic radical which is unsubstituted or substituted by a (C₁-C₃)-alkyl, by a hydroxyl or by a benzyl group; a 1-adamantylmethyl; an aromatic heterocycle unsubstituted, mono- or polysubstituted by a halogen, a (C₁-C₅)-alkyl, a (C₁-C₅)-alkoxy; a (C₁-C₃)-alkyl substituted by an aromatic heterocycle unsubstituted or mono- or polysubstituted by a halogen, a (C₁-C₅)-alkyl, a (C₁-C₅)-alkoxy, or else R²⁴ is hydrogen and R²⁵ is as defined above, or else R²⁴ and R²⁵ together with the nitrogen atom to which they are bonded, form a saturated 5-to 8-membered heterocyclic radical, said heterocyclic radical being other than morpholine when R³¹, R³², R³³, R³⁴ and R³⁵ and R⁴¹, R⁴², R⁴³, R⁴⁴ and R⁴⁵ are all hydrogen;
a group R²⁵ as defined above, when X is -(CH₂)ₘN(R²³)-; or
a group R²⁶, when X is a direct bond, R²⁶ being a (C₁-C₃)-alkyl; a (C₃-C₁₂)-cycloalkyl which is unsubstituted or substituted by a (C₁-C₅)-alkyl: a phenyl-(C₁-C₃)-alkyl which is unsubstituted or substituted by a halogen or by a (C₁-C₅)-alkyl; a cycloalkyl-(C₁-C₃)-alkyl in which the cycloalkyl is C₃-C₁₂ and is unsubstituted or substituted by a (C₁-C₅)-alkyl; or a 2-norbornylmethyl;
R³¹ R³² R³³, R³⁴ and R³⁵ and R⁴¹ R⁴², R⁴³, R⁴⁴ and R⁴⁵ are identical or different and are independently hydrogen, a chlorine, lodine or bromine atom, a (C₁-C₃)-alkyl, a (C₁-C₃)-alkoxy, a trifluormethyl, or a nitro group and R³³ is optionally a phenyl group,
R²² is hydrogen or a (C₁-C₃)-alkyl;
one of its salts.

18. Use according to claim 1 to 7 in which the Canabinoid Antagonist or inverse Agonist is selected from a compound of general formula II, 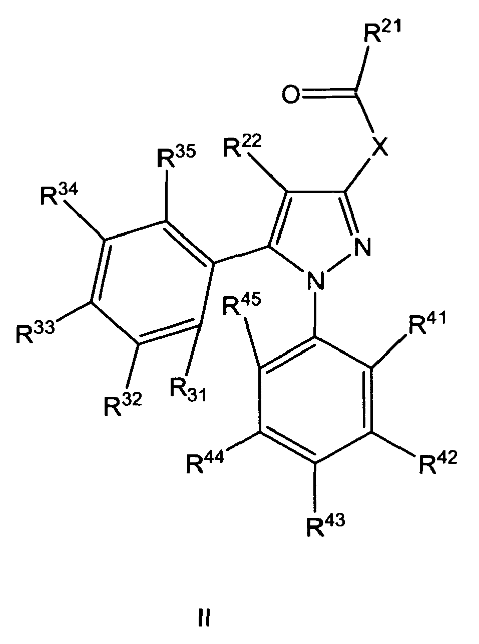 wherein
X is a direct bond or a group (-CH₂)ₘ-N(R²³) in which R²³ is hydrogen or a (C₁-C₃)-alkyl and m is 0 or 1; and
R²¹ is selected from
― NR²⁴R²⁵ in which R²⁴ is hydrogen and R²⁵ is phenyl, (C₁-C₈)-alkyl, or 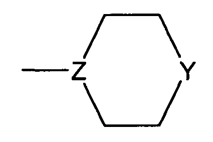 with Z being N or CH and Y being O, NR²⁷ or CHR²⁷, with R²⁷ being hydrogen or hydroxyl;
R³¹, R³², R³³, R³⁴ and R³⁵ and R⁴¹, R⁴², R⁴³, R⁴⁴ and R⁴⁵ are identical or different and are independently hydrogen, a halogen atom, a (C₁-C₈)-alkyl, a (C₁-C₃)-alkoxy, a trifluormethyl, or a nitro group and R³³ is optionally a phenyl group,
R²² being selected from hydrogen, CN or a (C₁-C₃)-alkyl; optionally in the form of a corresponding N-oxide, a corresponding salt or a corresponding solvate.

19. Use according to any of claim 17 or 18 in which the compound of general formula II is **characterized in that** R³¹, R³², R³⁴, R³⁵, R⁴², R⁴⁴ and R⁴⁵ are hydrogen.

20. Use according to claim 19 in which the compound of general formula II is **characterized in that** R³³, R⁴¹ and R⁴³ are selected from hydrogen, iodine, chlorine, bromine, (C₁-C₈)-alkyl or (C₁-C₃)-alkoxy.

21. Use according to claim 17 to 20 in which the compound of general formula II is selected from:
• 1-(2,4-dichlorophenyl)-5-(4-chlorophenyl)-4-methyl-N-(piperidin-1-yl)-1H-pyrazole-3-carboxamide;
• 1-(2,4-dichlorophenyl)-5-(4-bromophenyl)-4-methyl-N-(piperidin-1-yl)-1H-pyrazole-3-carboxamide;
• 1-(2,4-dichlorophenyl)-5-(4-chlorophenyl)-4-ethyl-N-(piperidin-1-yl)-1H-pyrazole-3-carboxamide;
• 1-(2,4-dichlorophenyl)-5-(4-bromophenyl)-4-ethyl-N-(piperidin-1-yl)-1H-pyrazole-3-carboxamide;
• 1-(2,4-dichlorophenyl)-5-(4-chlorophenyl)-N-(pipeddin-1-yl)-1H-pyrazole-3-carboxamide;
• 1-(2,4-dichlorophenyl)-5-(4-bromophenyl)-N-(piperidin-1-yl)-1H-pyrazole-3-carboxamide;
• 1-(2,4-dichlorophenyl)-5-(4-iodophenyl)-4-methyl-N-(piperidin-1-yl)-1 H-pyrazole-3-carboxamide; or
• 1-(2,4-dichlorophenyl)-5-(4-chlorophenyl)-N-(4-hydroxypiperidin-1-yl)-4-methyl-1 H-pyrazole-3-carboxamide;
optionally in the form of a corresponding N-oxide, a corresponding salt or a corresponding solvate.

22. Use according to any claim 1 to 7 in which the CB1 Antagonist or Inverse Agonist is selected from a compound; optionally in the form of a corresponding N-oxide, a corresponding salt or a corresponding solvate; identified through the following Reference table:
**Reference Table**
| **Literature (included by reference and included as pages** | **Page: in application (in article)** | **Figure No.: (Group No.)** | **Structure No.** | **Structure Code/Name** |
|---|---|---|---|---|
| **ANNEX 1:** | 73 (499) | 1 (I) | 1 | Rimonabant |
| Lange and Kruse, "Recent advances in CB1 cannabinoid receptor antagonists", Current Opinion in Drug Discovery & Development, 2004, 7(4): 498-506 | | | 2 | |
| | | | 3 | |
| | | | 4 | CP-272871 |
| | | | 5 | NIDA-41020 |
| | | | 6 | |
| | | | 7 | |
| | 74 (500) | 2 (II) | 8 | |
| | | | 9 | |
| | | | 10 | |
| | | | 11 | |
| | | | 12 | |
| | 75 (501) | 3 (III) | 13 | |
| | | | 14 | |
| | | | 15 | |
| | | | 16 | |
| | | | 17 | |
| | 76 (502) | 4 (IV) | 18 | |
| | | | 19 | |
| | | | 20 | |
| | | | 22 | |
| | | 5 (V) | 23 | SLV 319 |
| | | | 24 | SLV 326 |
| | | | 25 | |
| | | | 26 | |
| | | | 27 | |
| | 77 (503) | 6 (VI) | 28 | |
| | | | 29 | |
| | | | 30 | |
| | | | 31 | |
| | | | 32 | |
| | | 7 | 33 | |
| | | (VII) 35 | 34 | |
| | | | | |
| | 78 (504) | 8 (VIII) | 36 | O-1918 |
| **ANNEX 2:** | 85 (57) | 1 (IX) | **1** | Rimonabant |
| Smith and Fathi, "Recent advances in the research and development of CB1 antagonists", I Drugs 2005 8(1):53-66 | | | 2 | O-889 |
| | | | 3 | SR-147778 |
| | | | 4 | |
| | | | 6 | |
| | | | 7 | |
| | | 2 (X) | 8 | SLV-319 |
| | | | 9 | |
| | | | 10 | |
| | 86 (58) | 3 (XI) | 11 | |
| | | | 12 | |
| | | | 13 | |
| | | | 14 | |
| | | | 15 | |
| | | | 16 | |
| | | | 17 | |
| | | | 18 | |
| | 87 (59) | 4 (XII) | 19 | |
| | | | 20 | |
| | | | 21 | |
| | | | 22 | |
| | | | 23 | |
| | | | 24 | |
| | | | 25 | |
| | | 5 (XIII) | 26 | |
| | | | 27 | |
| | | | 28 | |
| | | | 29 | |
| | | 6 (XIV) | 30 | |
| | | | 31 | |
| | 88 (60) | 7 (XV) | 32 | |
| | | | 33 | |
| | | | 34 | |
| | | 8 (XVI | 35 | |
| | | | 36 | |
| | | | 37 | |
| | | | 38 | |
| | | | 39 | |
| | | 9 (XVII) | 40 | |
| | | | 41 | |
| | | | 42 | |
| | (89) 61 | 10 (XVIII) | 43 | |
| | | | 44 | |
| | | | 45 | |
| | | | 46 | |
| | | | 47 | |
| | | | 48 | |
| | | | 49 | |
| | | | 50 | |
| | | 11 (XIX) | 51 | |
| | | | 52 | |
| | | | 53 | |
| | | | 54 | |
| | | | 55 | |
| | | | 56 | |
| | | | 57 | |
| | | | 58 | |
| | (90) 62 | 12 (XX) | 59 | |
| | | | 60 | |
| | | | 61 | |
| | | | 62 | |
| | | | 63 | |

23. Use according to any claim 1 to 7 in which the CB1 Antagonist or Inverse Agonist is selected from the group of:
• 1,5-Diarylpyrazole cpds., preferably from close analogs to Group (I) according to the reference table in claim 22;
• tricyclic pyrazole cpds., preferably from close analogs to Group (II) according to the reference table in claim 22;
• tetracyclic pyrazole cpds., preferably from close analogs to Group (II) according to the reference table in claim 22;
• five-membered aromatic heterocyclic ring bioisostere cpds., preferably from close analogs to Group (III) according to the reference table in claim 22;
• six-membered aromatic heterocycle cpds., preferably from close analogs to Group (IV) according to the reference table in claim 22;
• 3,4-diarylpirazoline cpds., preferably from close analogs to Group (V) according to the reference table in claim 22;
• substituted amide cpds., preferably from close analogs to Group (VI) according to the reference table in claim 22;
• benzodioxole derivative cpds., preferably from close analogs to Group (VII) according to the reference table in claim 22;
• biphenyl derivative cpds., preferably from close analogs to Group (VIII) according to the reference table in claim 22;
• 1 ,5-Diaryl-pyrazole cpds., preferably from close analogs to Group (IX) according to the reference table in claim 22;
• 3,4-Diaryl-4,5-dihydropyrazole cpds., preferably from close analogs to Group (X) according to the reference table in claim 22;
• 4,5-Diaryl-imidazole cpds., preferably from close analogs to Group (XI) according to the reference table in claim 22;
• 1,2-Diaryl-imidazole cpds., preferably from close analogs to Group (XI) according to the reference table in claim 22;
• 2,3-Diaryl-1,2,4-triazole cpds., preferably from close analogs to Group (XII) according to the reference table in claim 22;
• Pyrrole cpds., preferably from close analogs to Group (XIII) according to the reference table in claim 22;
• Thiazole cpds., preferably from close analogs to Group (XIV) according to the reference table in claim 22;
• Imidazolidinedione cpds., preferably from close analogs to Group (XV) according to the reference table in claim 22;
• Six-membered-ring-core cpds., preferably from close analogs to Group (XVI) according to the reference table in claim 22;
• Four-membered-ring-core cpds., preferably from close analogs to Group (XVII) according to the reference table in claim 22:
• Bicyclic-core cpds., preferably from close analogs to Group (XVIII) according to the reference table in claim 22;
• Constrained tricyclic analog cpds., preferably from close analogs to Group (XIX) according to the reference table in claim 22;
• Constrained tetracyclic analog cpds., preferably from close analogs to Group (XIX) according to the reference table in claim 22; or
• Acyclic analog cpds., preferably from close analogs to Group (XX) according to the reference table in claim 22;
optionally in the form of a corresponding N-oxide, a corresponding salt or a corresponding solvate.
